# EUROPEAN PATENT SPECIFICATION

(11) **EP 4 476 741 B1**
(45) Date of publication and mention of the grant of the patent: **22.10.2025**
(21) Application number: 23703498.8
(22) Date of filing: 10.02.2023
(51) Int. Cl.: G16H 20/40, G16H 50/50, G16H 50/20

(54) **A METHOD AND SYSTEM FOR DETERMINING A MODEL OF A CARDIAC IMPLANT**
VERFAHREN UND SYSTEM ZUR BESTIMMUNG EINES MODELLS EINES HERZIMPLANTATS
PROCÉDÉ ET SYSTÈME DE DÉTERMINATION D'UN MODÈLE D'IMPLANT CARDIAQUE

(30) Priority: 10.02.2022 EP 22156210
(43) Date of publication of application: 18.12.2024
(73) Proprietor: FEops NV, 9052 Gent (BE)
(72) Inventor: DEBUSSCHERE, Nic, 9052 Gent (BE); OLIVEIRA, Vanda, 9052 Gent (BE); MORTIER, Peter Eddy J, 9052 Gent (BE)
(74) Representative: V.O.
(86) International application number: PCT/EP2023/053366
(87) International publication number: WO 2023/152326

(56) References cited:
- WO-A1-2019/179793
- US-A1- 2011 153 286
- US-A1- 2015 178 938

## Description

### FIELD OF THE INVENTION

The invention relates to a computer implemented method for determining a (deformed) model of a cardiac implant deployed in a cardiac region of a patient. The invention also relates to a system for determining a model of a cardiac implant deployable in a cardiac region. Also, the invention relates to a computer implemented method and a system for determining a model of a cardiac region. Furthermore, the invention relates to a computer program product. Additionally, the invention relates to the field of pre-operative planning of percutaneous, such as transcatheter, structural heart interventions, e.g. valve treatment such as valve implantation and/or repair, left atrial appendage occlusion, etc. Also, the invention relates to pre-operative prediction of the risk a patient developing abnormalities as a result of a structural heart intervention, such as transcatheter valve treatment.

### BACKGROUND TO THE INVENTION

Three dimensional models of a cardiac implant deployed in the cardiac region of a patient are often used for the purpose of pre-operative planning for the patient, selection of an optimum implant for the patient, and/or risk assessment for example prior to surgery. Various computer analysis techniques may be employed on said patient-specific models. For example, such a model may be a geometrical model based on which a meshed model is created for finite element modelling and computation. However, instead of finite element calculations, other kind of simulations are also possible, for instance involving computational fluid dynamics. The computational models of the cardiac implant in the cardiac region and/or the models of the cardiac region may also be used for multidisciplinary computational models, for instance involving fluid-structure interaction. It can be challenging to reconstruct patient-specific models based on images of the cardiac region (e.g. computer tomography images). Often, an image is obtained, comprising gaps whereby the gaps represent a lack of data.

Such models of the cardiac region may for example be used for pre-operative planning of heart interventions, such as valve treatment, left atrial appendage occlusion, etc. For example, an implant model representing a valve implant can be virtually deployed into a patient-specific anatomical model of the heart. In some examples, a library of implant models, each modeling geometrical properties of a corresponding valve implant, can be maintained. The implant models maintained in the library can be virtually deployed into the patient specific anatomical model of the heart valve to select one of the implant models for use in a percutaneous valve implantation procedure.

The valve treatment may for instance involve valve implantation and/or repair. For example, aortic (valve) stenosis is a pathology occurring when the aortic valve does not open fully because the leaflets calcify, thicken and stiffen and, as a result, the blood flow going from the heart to the systemic circulation decreases. Currently it is the most common valvular heart disease in the Western world and its prevalence is increasing with the aging population. The standard treatment for an aortic stenosis is the Surgical Aortic Valve Replacement (SAVR) aiming at reproducing the correct function of the native valve with an implanted valve. This invasive procedure typically requires total anesthesia, sternotomy (open-heart surgery) and cardiopulmonary bypass (the blood is pumped and oxygenated using an external machine). Undersizing of a valve implant may lead to paravalvular aortic regurgitation, while oversizing may result in a rupture of the aortic annulus or in a suboptimal functional behavior of the implant. Currently available planning tools provide insights into the patient anatomy and can, for example, be used to determine the size of the aortic annulus, or to measure the distance between the valve plane and the coronary ostia. A problem with these tools is that they do not provide preoperative insights into the interaction between a certain implant device and the specific patient anatomy, and can thus not be used to predict complications such as regurgitation. Such insights are extremely valuable for interventional cardiologists.

Trans-catheter aortic valve implantation (TAVI) or trans-catheter aortic valve replacement (TAVR) is a minimally-invasive procedure for treating aortic stenosis: (1) the valve (e.g. a bioprosthetic valve made of porcine pericardium sutured on a metal stent) is crimped inside a catheter, (2) the catheter is inserted,
for example, in the femoral artery, (3) pushed upstream along the aorta up to the aortic annulus and (4) the new valve is deployed within the diseased native valve. TAVI has the potential of treating high-risk patients and replacing the SAVR with a minimally-invasive intervention (no need for open-heart surgery or cardiopulmonary bypass) which can be performed in e.g. about 80 minutes. Main TAVI complications are vascular injury, stroke, cardiac injury (heart block, coronary obstruction, cardiac perforation), aortic regurgitation, cardiac conduction abnormalities and valve misplacement. Accurate pre-operative planning is crucial to select the optimal device size and to anticipate potential difficulties.

The heart intervention may also be related to other types of stent placement, medical device placement, etc. Various types of implants may be employed.

The known methods of building a model of an implant deployed in the cardiac region are typically computationally heavy, and may require an expert. This can result in waiting times of several hours or even days, before a clinician can obtain the results of the modelling of the deformed implant. Therefore there is a need for faster processing. There is a strong desire for being able to determine the model of the implant deployed in the cardiac region based on a patient-specific image of the cardiac region on-the-fly or in real-time.

WO 2019/179793 A1 discloses a system and method for selecting, from a series of cardiac implants having different sizes, the cardiac implant having optimum size for implantation in a patient. The method includes obtaining data representative of a patient-specific cardiac region and predicting the optimum size of the cardiac implant best matching a predefined criterion when deployed in the cardiac region. The predicting includes querying a database; determining parameter values for a parametric model representation of the patient-specific cardiac region; and/or entering the data representative of the patient-specific cardiac region into an artificial intelligence device.

### SUMMARY OF THE INVENTION

It is an object of the invention to provide for a method and a system that obviates at least one of the above mentioned drawbacks. In general, it is an objective to provide for a method and a system that allow efficient patient-specific pre-operative planning for the patient, patient-specific selection of an optimum implant, and/or patient-specific risk assessment prior to surgery.

Additionally or alternatively, it is an objective of the invention to improve the determination and/or reconstruction of a patient-specific (deformed) model of an implant deployed in a cardiac region based on a received patient-specific image of said cardiac region.
Additionally or alternatively, it is an objective of the invention to improve the efficiency of obtaining a patient-specific model of the cardiac implant based on a received patient-specific image of said cardiac region, preferably enabling on-the-fly or real-time determination of said model.

Additionally or alternatively, it is an object to obtain a more robust method and system for building a patient-specific model of a cardiac implant and/or a cardiac region based on one or more images of said cardiac region.

Additionally or alternatively, it is an object of the invention to improve the quality and/or accuracy of determining a patient-specific model of a cardiac region.

Thereto, the invention provides for a computer implemented method for determining a model of a cardiac implant deployed in a cardiac region of a patient. The method includes providing a database including a plurality of different records, each record including at least data representative of an image of a cardiac region and an associated model of the cardiac implant deployed in said cardiac region. The method includes receiving a patient-specific image of the cardiac region for which the model of the cardiac implant deployed in said cardiac region is to be determined. The method includes calculating an approximation of the patient-specific image of the cardiac region based on a weighted combination of images of the cardiac region in a plurality of records. The approximation is performed by means of an approximation function which is configured to approximate the patient-specific image of the cardiac region based on the weighted sum of data indicative of images of the cardiac region in a plurality of records. The method includes determining the model of the cardiac implant deployed in the cardiac region associated to the patient-specific image of said cardiac region based on parameters of the approximation function. Hence, in this, or another, way the method includes determining the model of the cardiac implant deployed in the cardiac region associated to the patient-specific image of said cardiac region based on a weighted combination of models of the cardiac implant associated to the images of the cardiac region in the plurality of records used for approximating said patient-specific image of the cardiac region.

The database can comprise data indicative of a plurality of patients. The data includes for each patient data indicative of the cardiac region, and data indicative of the shape of a deformed implant deployed in said cardiac region. A newly received (optionally processed) patient-specific image of the cardiac region can be approximated by a weighted combination of images of the cardiac region of multiple patients in the database. Advantageously, based on (e.g. the same) weighted combination, applied on models of deployed implants for the multiple patients, a novel model of the deployed cardiac implant linked to the newly received (optionally processed) patient-specific image can be determined/built. This provides a highly robust, efficient and cost-effective way of obtaining a patient-specific model of the cardiac implant deployed in the cardiac region of the (new) patient.

The method provides for an accurate real time modelling of the cardiac implant deployed in the cardiac region using the patient-specific (e.g. three-dimensional) image of the cardiac region as basis. For this purpose, a weighted combination of data indicative of models of the (deformed) cardiac implant in the plurality of records is used. The constructed model of the deployed cardiac implant can be used for various applications. The effect of inserting said cardiac member inside the heart of the patient can be predicted. Additionally or alternatively, an adequate positioning, arrangement, and/or size of said cardiac member can be easily selected. The method provides for an efficient way of obtaining the (computational) model of the cardiac implant deployable in the patient-specific cardiac region represented by the image of said cardiac region. It will be appreciated that the image of the patient-specific cardiac region may be (pre-)processed. This may involve, segmentation, subtracting a mean/average image (of images in multiple records in the database), etc.

Instead of merely selecting one cardiac implant model in a database, based on similarities (e.g. closest fit), the model is calculated/built based on a weighted combination of other models of the cardiac implant in a plurality of records in the database. How the models of the (deformed) cardiac implant deployed in the cardiac region of different patients are combined is determined by first approximating (data indicative of) the newly received patient-specific image of the cardiac region based on a combination of (data indicative of) images of the cardiac region in multiple records of the database (cf. images for other patients). The newly received patient-specific image may be first (pre-)processed. This pre-processing may for example include subtracting the average/mean image of the multiple images of the cardiac region in the multiple records of the database from said newly received patient-specific image. Such a (pre-)processing may significantly improve the approximation, and thus the weighted combination. As a result, the weighted combination used for determining the model of the cardiac implant deployed in the cardiac region can also be significantly improved. Additionally or alternatively, one or more other (pre-)processing steps may be carried out.

The reconstructed/approximated patient-specific image may be a function of images in multiple records in the database. However, some records may have a larger contribution (i.e. larger weight) and some records may have a smaller contribution (i.e. smaller weight). Based on said contributions, the weights can be calculated for determining an adequate combination for accurately reconstructing or approximating the patient-specific image. Advantageously, the weighted combination or data based on said weighted combination, can then subsequently be used for determining the (computer) model of the cardiac implant associated to said patient-specific image. In some examples, the same weighted combination is used for the associated models for determining/building the novel model of the (deformed) cardiac implant deployed in the cardiac region.

Various computational techniques may be used for determining said combinations. The computational technique may be based on an optimization algorithm. The algorithm may be configured to find a combination which minimizes the difference between the image based on the combination of images and the actual image. In some examples, a statistical model is used for determining the combination. Optionally, a trained machine learning model, such as a trained neural network, is used for determining the combination.

It will be appreciated that the determined model of the cardiac implant deployed in the cardiac region associated to the patient-specific image of said cardiac region may provide for a prediction of the deployment of a real cardiac implant. The determined model can thus be used for predicting what the actual cardiac implant deployed in said cardiac region of the patient will look like. Advantageously, this can be performed efficiently using the received (optionally (pre-)processed) image of the cardiac region of the particular patient, and data stored in the database. The model can be built or predicted substantially faster than other computational methods, for instance involving finite element analysis. The deployed form of the actual cardiac implant (e.g. bent shape in order to fit in the cardiac region) can be efficiently predicted/estimated by the model according to the method of the disclosure. This prediction of the actual deployed implant in the specific patient provided by the determined model can be used for pre-operative planning for the patient, selection of an optimum implant for the patient, and/or risk assessment (for example) prior to surgery.

The models in the plurality of records of the database used for determining the model of the cardiac implant deployed in the cardiac region associated to the received patient-specific image may relate to the same base implant or starting implant. This allows for an accurate model determination/prediction (e.g. for a new patient) using the weighted combination of the models in the plurality of records. However, the method and system according to the disclosure may be used for determining different models relating to different model types, models with different shapes, sizes, orientation, states, settings and/or arrangements, or the like, if such relevant data is available in the database. For example, the method and system according to the disclosure can be used for determining models of different cardiac implants and/or different cardiac implant configurations. On the basis of the determined/built models, the most suitable cardiac implant may be selected, for example at least partially in view of pre-operative planning for the patient, selection of an optimum implant for the patient, and/or surgery risk assessment.

Optionally, the images are three-dimensional images.

Optionally, the models are three-dimensional models.

The patient-specific image of the cardiac region and the images in the multiple records of the database may for instance be based on a computer topography (CT) image. However, other types of images can also be used, such as for instance magnetic resonance imaging (MRI) or positron emission tomography. Hybrid imaging techniques may also be used, such as PET-MRI, PET-CT, etc. It is also envisaged to use three-dimensional imaging obtained by means of data fusion. It will be appreciated that the images of the cardiac region may also be based on 2D images. Various medical imaging techniques may be used. The captured images may be (pre-)processed (e.g. segmentation, data harmonization, statistical processing such as subtracting an average/mean image, etc.).

Optionally, the model is used for predicting a deployment configuration for implantation of an implant in a patient, wherein the deployment configuration includes size, position and/or type of the cardiac implant matching a predefined criterion when deployed in the cardiac region of the patient.

The predicting can include querying a database including a plurality of records, each record including data representative of an image of a cardiac region and an associated size and/or position, of the cardiac implant of the series in the cardiac region, e.g. as a result of cardiac implant deployment and deformation of the cardiac region. The record can include data representative of (an implant model representing) a cardiac implant of a predetermined size. The predicting can include determining parameter values for a parametric model representation of the patient-specific cardiac region. A second parametric model predicts the optimum size and/or optimum position, of a cardiac implant of the series in the cardiac region, e.g. as a result of cardiac implant deployment and deformation of the cardiac region associated therewith, on the basis of the parameter values.

Optionally, the weighted combination is a linear combination.

The model of the cardiac implant deployed in the cardiac region may be determined by performing a linear combination of different models of cardiac implants deployed in different cardiac regions (different patients, different cardiac region (e.g. of same patient), etc.), each having an assigned weight (cf. contribution). With other words, the model can be calculated as a linear function of other models in the database. In this way, if the database has sufficient variety in available data stored in the plurality of records, the model associated to the patient-specific image (e.g. based on a three-dimensional image) can be determined accurately and efficiently. A real-time determination can be performed in a cost-effective way.

At patient level, data of a new patient can be approximated by means of a weighted combination of data of existing patients in the database. In this way, the newly received image of the cardiac region, which may undergo one or more pre-processing steps (e.g. segmentation, subtracting mean/average image of multiple images in records of the database, etc.), can be approximated as a weighted combination of images of the plurality of records in the database. Subsequently, advantageously, a combination based on the determined weighted combination can be used for obtaining a model of the cardiac implant deployed in the cardiac region. The model may be deformed as it represents a deployed cardiac implant in the cardiac region.

The weighted combination can be effectively applied as the model of the implant provides for uniform data, which does not differ for different patients. In other words, the undeformed implant can be the same for different patients (cf. records in the database), whilst the deformed implant deployed in the patient-specific cardiac region can be different for different patients.

Optionally, a mean/average image is determined based on the (pre-processed) images in the multiple records in the database.

Optionally, the received patient-specific image is a preprocessed image obtained by subtracting the mean/average image from said received patient-specific image, wherein the resulting patient-specific image is used for calculating the approximation of the patient-specific image of the cardiac region based on a weighted combination of images of the cardiac region in a plurality of records. Optionally, subsequently, the mean/average image is added to the approximated image.

Optionally, the approximation of the patient-specific image of the cardiac region is based on the weighted combination of images of the cardiac region of a selected subset of records.

In some examples, the corresponding model for the (data indicative of the) patient-specific image is built based on a combination of selected models of selected subset of records. The models in the subset of records may be selected based on similarity. The models in said subset may enable a sufficiently accurate prediction of the model associated to the received patient-specific image.

Optionally, the subset of records is selected based on its weighted contribution for approximation of the image of the cardiac region.

In some examples, only models which have a relative high contribution for approximating the 3D image of the cardiac region are used for determining/predicting the model.

Optionally, the approximation of the patient-specific image of the cardiac region is based on the weighted combination of images of the cardiac region of all the records in the database.

More similar data in the database may be assigned a larger weight in the weighted combination, whilst less similar data may be assigned a smaller weight in the weighted combination. The reconstructed/approximated patient-specific image may be a function of 3D images in all the records in the database.

In some examples, the approximation function is a linear function, with a plurality of terms, each term being associated to one image of the plurality of records. Furthermore, each term may have a weight parameter, the weight parameter determining the impact of the term on the output of the approximation function. An accurate approximation of the (data indicative of the) received patient-specific image may be obtained using data in multiple records of the database (e.g. data associated to different patients). Based on the weighted combination, a similar weighted combination of existing models in the records can be used for building/reconstructing a model of the cardiac implant deployed in the cardiac region, the model linked to the received (data indicative of) the patient-specific image of the cardiac region.

Optionally, each record includes at least one model of the cardiac region with a cardiac implant deployed in the cardiac region, wherein the model of the cardiac region with implant associated to the patient-specific image of the cardiac region is determined based on weighted combination of models with cardiac implant associated to images of cardiac regions in the plurality of records used for approximating the patient-specific image of the cardiac region.

Additionally or alternatively, also a model of the cardiac region can be built with one or more implants therein. In this way, a highly efficient analysis of the impact of the cardiac implants can be obtained.

Optionally, the models of the cardiac implant in the different records represent the same cardiac implant deployed in cardiac regions of different patients, wherein an undeformed cardiac implant is identical for all the records.

The deformed cardiac implants, resulting from deployment in the cardiac region of the unique patients, may be different. As a result, the models of the cardiac implant associated to the images of the cardiac region in the plurality of records used for approximating said patient-specific image of the cardiac region, may be different (cf. different deformations due to deployment).

It will be appreciated that an implant may be a structure which is to be provided or implanted in the cardiac region of a patient.

Optionally, the cardiac implant is at least one of a heart valve, a stent or an implantable medical device. The implant may be various structures implantable in the cardiac region, such as for instance a stent valve or a left atrial appendage occlusion device. A wide variety of other (implantable) structures can be employed.

Various cardiac implants can be employed. It is also envisaged that the records in the database have various models with different types of implants.

Optionally, the determined model of the cardiac implant deployed in the cardiac region is stored in the database and usable for determining subsequent patient-specific cardiac implant models.

In some examples, a distinction is made between predicted/reconstructed cardiac implant models and "ground-truth" cardiac implant models which have been built, adapted and/or reviewed by a human expert. For example, in some examples, only ground-truth models are used for building/reconstructing new models. However, in some examples, also previously predicted/reconstructed models of the cardiac implant can be used for future predicting/reconstruction of cardiac implant models.

Optionally, the models are at least one of a computer model, a geometrical model, a meshed model or a parametric model.

Various types of models can be used. A geometrical model may have geometrical data of the cardiac region. A meshed model may be a finite element model, finite volume model, or other types of meshed models which can be used for computational analysis. A parametric model may have a set of parameters based on which the 3D model can be reconstructed.

Optionally, each record in the database is associated to a patient.

Optionally, the images of the cardiac region are pre-processed, wherein the pre-processing includes segmentation.

The images may be patient-specific medical images. For example, CT images or the like may be used. In some examples, the images are three-dimensional images (e.g. accumulation of 2D images). The images may be pre-processed in order to obtain a segmented image or a mask. A distinction can be made between pixels/voxels which are inside an anatomy of the patient and pixels/voxels which are outside the cardiac region of interest of the patient. In some examples, all images in records of the database are pre-processed. It will be appreciated that the database may include both the unprocessed image and the pre-processed image in a same record.

Optionally, pre-processing includes automatic segmentation. In some examples, a trained machine learning model is used for performing the automatic segmentation. However, other techniques may also be used, for instance the segmentation may be performed procedurally.

Optionally, pre-processing includes manual segmentation.

According to an aspect, the invention provides for a system for determining a model of a cardiac implant deployable in a cardiac region cardiac region. The system includes a database comprising a plurality of different records, each record including data representative of an image of a cardiac region and an associated model of the cardiac implant deployed in said cardiac region The system includes a processing unit configured to receive a patient-specific image of the cardiac region for which the model of the cardiac implant deployed in said cardiac region is to be determined. The processing unit is configured to calculate an approximation of the patient-specific image of the cardiac region based on a weighted combination of images of the cardiac region in a plurality of records. The approximation is performed by means of an approximation function which is configured to approximate the patient-specific image of the cardiac region based on the weighted sum of data indicative of images of the cardiac region in a plurality of records. The processing unit is configured to determine the model of the cardiac implant deployed in the cardiac region associated to the patient-specific image of said cardiac region based on parameters of the approximation function. Hence, in this, or another, way the processing unit can determine the (deformed) model of the cardiac implant deployable in the cardiac region associated to the patient-specific image of said cardiac region based on weighted combination of models of the cardiac implant associated to the images of the cardiac region in the plurality of records used for approximating said patient-specific image of the cardiac region.

Advantageously, data of a new patient can be reconstructed using a combination of data of other patients in the records of the database. In this way, models of cardiac implants can be determined very efficiently. In some cases, the models can be determined on-the-fly or in real-time. If the database includes sufficient records, a highly accurate and robust approximation can be made. Additionally, the approximation can be more predictable and more reliable for use for medical purposes compared to statistical learning methods. For example, in black-box statistical methods (e.g. trained neural network model), it is often not clear how the output models are generated. In some cases, the use of black-box or grey-box models may thus not be suitable for use for various medical applications. The system according to the disclosure, can provide more reliable outputs.

According to an aspect, the invention provides for a computer implemented method for determining a model of a cardiac region, the method including: providing a database including a plurality of different records, each record including at least data representative of an image of a cardiac region and an associated model of said cardiac region; receiving a patient-specific image of the cardiac region for which the model of the cardiac region is to be determined; calculating an approximation of the patient-specific image of the cardiac region based on a weighted combination of images of the cardiac region in a plurality of records; and determining the model of the cardiac region associated to the patient-specific image of the cardiac region based on weighted combination of models associated to the images of the cardiac region in the plurality of records used for approximating said patient-specific image of the cardiac region.

Advantageously, the method provides for an accurate and real time modelling of the cardiac region using the patient-specific three-dimensional image of the cardiac region. The constructed model can be used for various application, such as for instance artificial cardiac member (e.g. valve, stent, device, etc.) modelling. The effect of inserting said cardiac member inside the heart of the patient can be assessed. Additionally or alternatively, an adequate positioning, arrangement, and/or size of said cardiac member can be easily selected. The method provides for an efficient way of obtaining the (computational) model of the cardiac region.

Instead of merely selecting one heart model in a database, based on similarities (cf. closest fit), the model is calculated based on a combination of other models in the database. How the models are combined is determined by first approximating the patient-specific 3D image based on a combination of 3D images in the database (cf. images for other patients).

The reconstructed/approximated patient-specific image may be a function of 3D images in multiple records in the database. However, some records may have a larger contribution (cf. weight) and some records may have a smaller contribution (cf. weight). Based on said contributions, the weights can be calculated for determining an adequate combination for accurately reconstructing or approximating the patient-specific image. Advantageously, this information can then subsequently be used for determining the computer model associated to said patient-specific image.

Various computational techniques may be used for determining said combinations. The computational technique may be based on an optimization algorithm. The algorithm may be configured to find a combination which minimizes the difference between the image based on the combination of images and the actual image. In some examples, a statistical model is used for determining the combination. Optionally, a trained machine learning model, such as a trained neural network, is used for determining the combination.

The patient-specific image may for instance be a computer topography (CT image). However, other types of images can also be used, such as for instance magnetic resonance imaging (MRI) or positron emission tomography. Hybrid imaging techniques may also be used, such as PET-MRI, PET-CT, etc. It is also envisaged to use three-dimensional imaging obtained by means of data fusion.

Optionally, the model is used for predicting a deployment configuration for implantation of an implant in a patient, wherein the deployment configuration includes size, position and/or type of the cardiac implant matching a predefined criterion when deployed in the cardiac region of the patient.

The method may include obtaining data representative of a series of implant models representing a series of cardiac implants having different sizes. Such data can include a three-dimensional model, such as a computer aided design (CAD) model or a finite element model. The method may include predicting an optimum size, and additionally or alternatively an optimum position, of the cardiac implant best matching a predefined criterion when deployed in the cardiac region. Determining the optimum size and/or position, can take into account cardiac implant deployment and deformation of the cardiac region. The predefined criterion can be a lowest risk of complications during and/or after deployment of the actual implant in the actual cardiac region of the patient. The complications can e.g. be mechanical complications (such as stress, strain, mechanical pressure, contact area), electrical complications (such as electrical conduction problems), hydraulical complications (such as blood flow, leakage, regurgitation), risk of misplacement of the implant, or the like. The criterion can e.g. be associated with optimum interaction between the implant and the cardiac region. The interaction can be at least one of mechanical interaction, such as contact pressure, strain, contact area; leakage; regurgitation; cardiac conduction abnormalities; risk of implant misplacement; or the like.

It is also possible that the predefined criterion is a best overall match of the cardiac implant size for the given cardiac region. Especially when comparing large numbers of clinical data of previous cardiac implants in cardiac regions, for a given cardiac region a best average size, and optionally position, can be determined in view of all potential complications encountered in the clinical data.

The predicting can include querying a database including a plurality of records, each record including data representative of a three-dimensional image of a cardiac region and an associated size and/or position, of the cardiac implant of the series in the cardiac region, e.g. as a result of cardiac implant deployment and deformation of the cardiac region. The record can include data representative of (an implant model representing) a cardiac implant of a predetermined size. Thus, different records may apply to differently sized cardiac implants. The predicting can include determining parameter values for a parametric model representation of the patient-specific cardiac region. A second parametric model predicts the optimum size and/or optimum position, of a cardiac implant of the series in the cardiac region, e.g. as a result of cardiac implant deployment and deformation of the cardiac region associated therewith, on the basis of the parameter values.

Optionally, each record comprises at least a model of the cardiac region without a cardiac implant, and a model of the cardiac region with a cardiac implant.

For the received patient-specific image for which a corresponding (computer) model is to be determined, models with cardiac implants in multiple records can be used for building/reconstructing a model with cardiac implant linked to the received patient-specific image, and/or models without cardiac implants in multiple records can be used for building/reconstructing a model without cardiac implant linked to the received patient-specific image.

Optionally, the cardiac region includes at least a portion of the heart.

The cardiac region may also include a portion adjacent to the heart. It is also possible that the model of the cardiac region is associated to a selected/filtered region within the actual cardiac region of the patient. For example, the model may relate to only heart chambers, arteries or other parts of the cardiac region.

According to an aspect, the invention provides for a computer program product configured for performing, when run on a controller, the steps of the method according to the disclosure.

According to an aspect, the invention relates to a method of predicting a deployment configuration for implantation of an implant in a patient.

Optionally, the deployment configuration includes size, position and/or type of the cardiac implant matching a predefined criterion when deployed in the cardiac region of the patient, wherein the model of the cardiac implant is determined according to the method of the disclosure.

Optionally, the predefined criterion is a lowest risk of complications during and/or after deployment of the actual implant in the actual cardiac region of the patient.

Optionally, data representative of a size and/or type, of cardiac implant to be implanted in the cardiac region of the patient is obtained using the model determined according to the method of the disclosure.

The method provides the advantage that the size of the cardiac implant best matching the predefined criterion when deployed in the cardiac region can be predicted.

Optionally, when multiple types of implant are available, the method allows predicting the type of the cardiac implant best matching the predefined criterion when deployed in the cardiac region.

Optionally, the model determined according to the disclosure is used for predicting an interaction between the cardiac implant and cardiac region.

Optionally, the prediction of the optimum size, and optionally optimum position, of a cardiac implant of the series is output.

According to an aspect, the invention relates to a method for pre-operative planning of percutaneous structural heart interventions, wherein the model determined according to the method of the disclosure is used for performing the pre-operative planning.

According to an aspect, the invention relates to pre-operative prediction of the risk a patient developing abnormalities as a result of for example transcatheter valve treatment, left atrial appendage occlusion, etc., wherein the prediction of the risk is calculated using the model determined according to the method of the disclosure.

Optionally, when multiple types of implant are available at least some of which have multiple sizes, the method allows predicting the size and type of the cardiac implant best matching the predefined criterion when deployed in the cardiac region. Optionally, the method allows predicting the implant position of the cardiac implant best matching the predefined criterion when deployed in the cardiac region.

"Optional" or "optionally" means that the subsequently described event or circumstance may or may not occur, and that the description includes instances where said event or circumstance occurs and instances where it does not.

It will be appreciated that a deep learning neural network can be seen as a representation-learning method with a plurality of levels of representation, which can be obtained by composing simple but non-linear modules that each transform the representation at one level, starting with the raw input, into a representation at a higher, slightly more abstract level. The neural network may identify patterns which are difficult to see using conventional or classical methods. Hence, instead of writing custom code specific to a problem of identifying visual patterns in the images of the fecal sample of the subject, the network can be trained to be able to handle different and/or changing images of the feces sample e.g. using a classification algorithm. Training data may be fed to the neural network such that it can determine a classification logic for efficiently analyzing intestinal microflora of the subject.

It will be appreciated that any of the aspects, features and options described in view of the computer implemented methods apply equally to the systems and the described other methods, and computer program products. It will also be clear that any one or more of the above aspects, features and options can be combined.

### BRIEF DESCRIPTION OF THE DRAWING

The invention will further be elucidated on the basis of exemplary embodiments which are represented in a drawing. The exemplary embodiments are given by way of non-limitative illustration. It is noted that the figures are only schematic representations of embodiments of the invention that are given by way of non-limiting example.

In the drawing:
Fig. 1 shows a schematic diagram of an embodiment of a method;
Fig. 2 shows a schematic diagram of an embodiment of a cardiac region and cardiac implant;
Fig. 3 shows a schematic diagram of an embodiment of exemplary images and models;
Fig. 4 shows a schematic diagram of an embodiment of an exemplary patient-specific image of a cardiac region;
Fig. 5 shows a schematic diagram of an embodiment of (pre-)processing of cardiac region images;
Fig. 6 shows a schematic diagram of an embodiment of an approximation of a patient-specific image;
Fig. 7 shows a schematic diagram of an embodiment of a determination of a patient-specific model of the cardiac implant;
Fig. 8 shows a schematic diagram of comparative results;
Fig. 9 shows a schematic diagram of an embodiment of a system; and
Fig. 10 shows a schematic diagram of a method.

### DETAILED DESCRIPTION

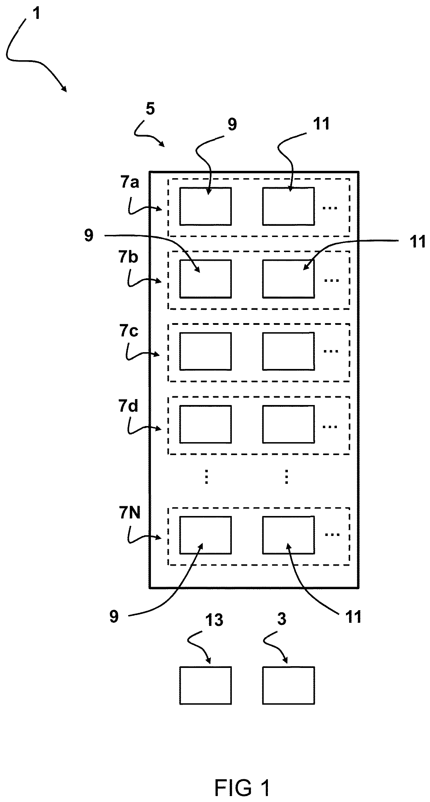

Fig. 1 shows a schematic diagram of an embodiment of a computer implemented method 1 for determining a model 3 of a cardiac implant deployed in a cardiac region of a patient. The method includes providing a database 5 including a plurality of different records 7a-7N, each record including at least data representative of an image 9 of a cardiac region and an associated model 11 of the cardiac implant deployed in said cardiac region. A patient-specific image 13 is received of the cardiac region for which the model 3 of the cardiac implant deployed in said cardiac region is to be determined. The method includes calculating an approximation of the patient-specific image 13 of the cardiac region based on a weighted combination of images 9 of the cardiac region in a plurality of records 7a-7N. Furthermore, the method includes determining the model 3 of the cardiac implant deployed in the cardiac region associated to the patient-specific image 13 of said cardiac region based on weighted combination of models 11 of the cardiac implant associated to the images of the cardiac region in the plurality of records 7a-7N used for approximating said patient-specific image 13 of the cardiac region.

Based on the weighted combination used for approximating the (optionally processed) patient-specific image, the deformed shape of the implant can be predicted/calculated. The weight factors in the weighted combination can be applied to the implant, and a deformed shape of the implant can be efficiently and accurately determined/predicted without requiring computationally intensive analysis (e.g. finite element analysis). The obtained model of the implant can be superimposed on the patient-specific anatomy of the cardiac region. In some examples, the patient-specific anatomy of the cardiac region is fit around the deformed implant. In this way, also a model of the cardiac region can be obtained. In some examples, the method can also be used to predict deformation of the patient-specific anatomy.

Optionally, all images of the cardiac region in the records in the database, and the newly received patient-specific image of the cardiac region for which the model of the cardiac implant deployed in said cardiac region is to be determined, are pre-processed. One or more pre-processing steps may have been performed for all images of the cardiac region in the records in the database, and for the new patient-specific image of the cardiac region. The resulting pre-processed images may be segmented images or masks.

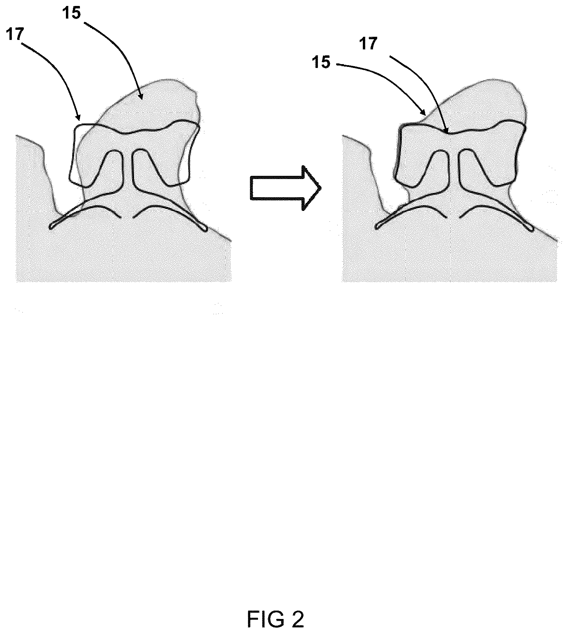

Fig. 2 shows a schematic diagram of an embodiment of a cardiac region 15 and an exemplary model of a cardiac implant 17 deployed in said cardiac region. The cardiac region can be a segmented image of a target implementation area of a patient. The image may be segmented to include features corresponding to relevant patient anatomy. The model of the cardiac implant 17 by performing the method according to the disclosure. The determined deformed cardiac implant 17 deployed in the cardiac region can be used to estimate a deformed cardiac region of the patient. For example, the cardiac region may be fit to suitably surround the model of the cardiac implant 17 at the contact regions. In this way, also a fast indication of the deformed cardiac region can be determined based on the deformed cardiac implant 17. Advantageously, the determination of the model of the cardiac implant 17 and the optional fitting of the cardiac region based on said determined model of the cardiac implant 17 can be carried out efficiently. The results can be obtained on-the-fly or in real-time.

The deformation of the cardiac implant deployed in the patient-specific cardiac region can be calculated using weight parameters of the weighted combination for approximation of the patient-specific image of the cardiac region based on other (patient-specific) images in (patient) records of the database. Additionally or alternatively, in some examples, it is possible to calculate a model of the deformed anatomy of the cardiac region using said weight parameters. Additionally or alternatively, a model is determined based on weighted combination of models associated to the images of the cardiac region in the plurality of records used for approximating said patient-specific image of the cardiac region, wherein the model includes the cardiac implant and at least a portion of the cardiac region (e.g. cardiac region surrounding the cardiac implant).

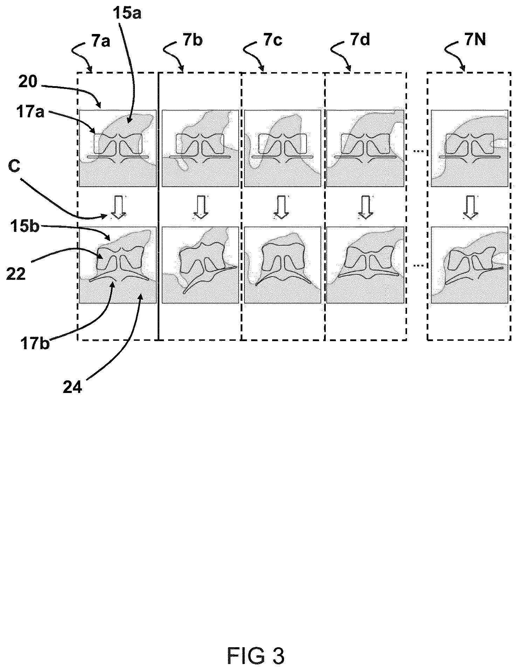

Fig. 3 shows a schematic diagram of an embodiment of exemplary images of cardiac regions and associated models of the cardiac implant deployed in said respective cardiac regions. The models of the deformed deployed cardiac implants in the cardiac regions may be determined by means of computational simulations C (e.g. physics simulations, finite element simulation, etc.) based on the image of the cardiac region and characteristics of the cardiac implant to be deployed in said cardiac region. The data may be stored in different records 7a-7N in the database. The database may include multiple (patient-specific) records, each record including a (optionally pre-processed) image 20 of the cardiac region without a deployed cardiac implant. For the sake of illustration an undeployed (i.e. undeformed) cardiac implant 17a is illustrated overlapped with the image 20 of the cardiac region. However, this illustrative undeformed cardiac implant 17a is not present in the image 20 of the cardiac region, but optionally it may be taken into account. Furthermore, each record includes a model 22 of a deployed cardiac implant 17b in the cardiac region. The deployed cardiac implant 17 is deformed as a result of this deployment (interaction with the cardiac region anatomy of the patient). Furthermore, optionally, the record also includes a model 24 of a deformed cardiac region with the deployed cardiac implant therein. It is possible to use separate models, or one model including both the deployed cardiac implant and the deformed cardiac region. The model may be two-dimensional or three-dimensional. The data in the database can be used for efficiently determining a model of a cardiac implant deployed in a cardiac region of a new patient, without requiring for time-consuming simulations. More particularly, a new patient-specific image may be received of the cardiac region for which the model of the cardiac implant deployed in said cardiac region is to be determined. Next, an approximation of the patient-specific image of the cardiac region may be calculated based on a weighted combination of images of the cardiac region in a plurality of records. Then, the model of the cardiac implant deployed in the cardiac region associated to the received patient-specific image of said cardiac region of the new patient can be determined based on weighted combination of models of the cardiac implant associated to the images of the cardiac region in the plurality of records used for approximating said patient-specific image of the cardiac region.

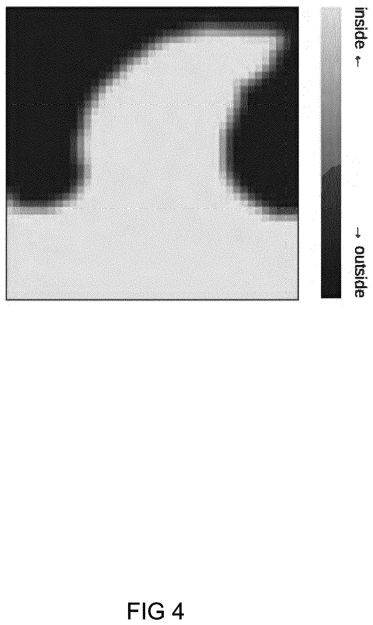

Fig. 4 shows a schematic diagram of an embodiment of an exemplary patient-specific image of a cardiac region. In this example, an image of the cardiac region obtained by means of a medical imaging unit (e.g. CT system) is pre-processed. The pre-processing includes segmenting the image in order to obtain a segmented image of the cardiac region. The segmented image or mask image of the cardiac region can be cropped around a target implantation area.

A segmentation has been applied to the image of an anatomy of the cardiac region of the patient. However, additionally or alternatively, various other pre-processing steps may be performed. For example, subtracting an average/mean image from the newly received patient-specific image may result in an improved weighted combination. Afterwards, the average/mean image may be added back to the approximated image. Similarly, an average/mean model of the cardiac implant can be calculated an subtracted from the models in the different records. Afterwards, the average/mean model may be added back to the approximated model.

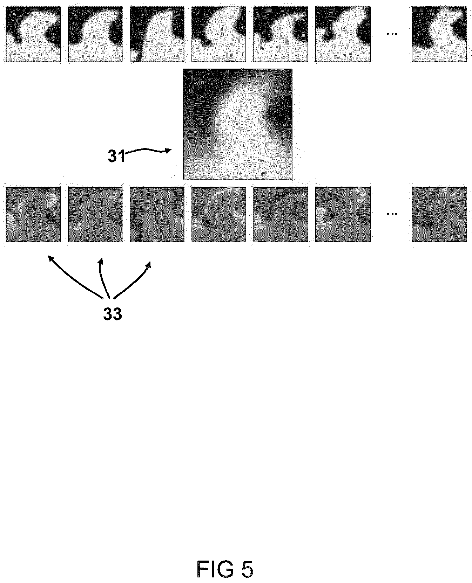

Fig. 5 shows a schematic diagram of an embodiment of (pre-)processing of images of the cardiac region. In this example, segmented images of the cardiac regions in the plurality of records are used for determining a mean/average image 31 of the cardiac region. The (pre-)processing includes subtracting the mean/average image. In this way, the resulting images 33 of the cardiac region indicate the deviation from the mean/average.

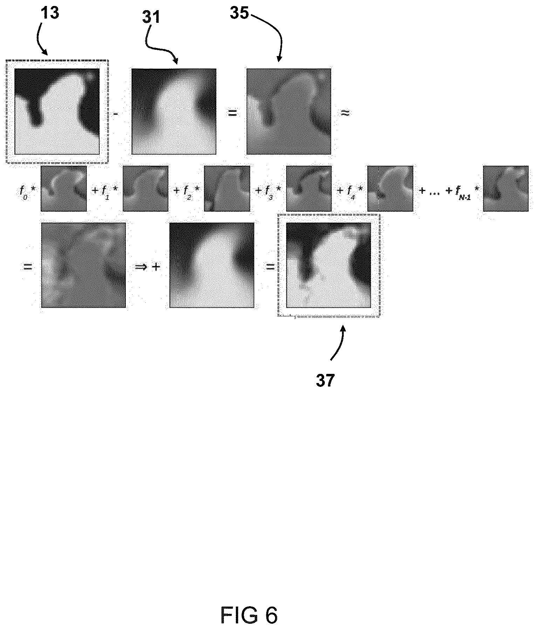

Fig. 6 shows a schematic diagram of an embodiment of an approximation of a patient-specific image 35. The patient-specific image 35 is (pre-)processed by subtracting the mean/average image 31 from an initial segmented image 13. This received patient-specific image 35 for which the model of the cardiac implant deployed in said cardiac region is to be determined can be used for calculating an approximation based on a weighted combination of (pre-)processed images of the cardiac region in a plurality of records. The weights factors are indicated by f₀-f_{(N-1)}. Optionally, the mean/average image 31 can be added to the approximation as a post-processing step in order to obtain the approximated image 37.

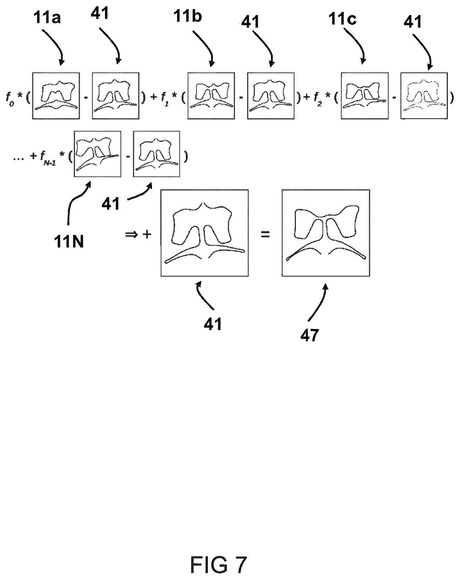

Fig. 7 shows a schematic diagram of an embodiment of a determination of a patient-specific model of the cardiac implant. The model of the cardiac implant deployed in the cardiac region associated to the patient-specific image 35 of said cardiac region can be determined based on weighted combination of models 11a-11N of the cardiac implant associated to the images of the cardiac region in the plurality of records used for approximating said patient-specific image of the cardiac region. Advantageously, the weight factors f₀-f(_{N-1}) can be used in the weighted combination of the relevant models. In this example, the cardiac implant model with mean/average deformation is optionally subtracted from the deformed models (i.e. pre-processing), in order to enhance the results. This mean/average deformation of the cardiac implant can be added to the approximated model in order to obtain the estimation of the actual cardiac implant deformation 47.

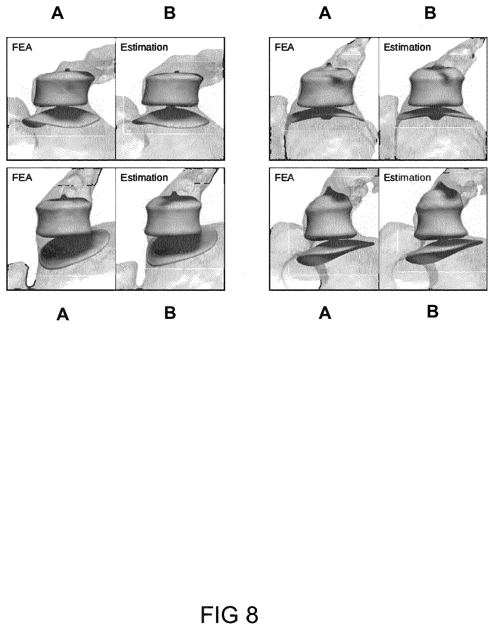

Fig. 8 shows a schematic diagram of comparative results. The left side A of each example illustrates results obtained by performing computational analysis, namely exemplary finite element analysis. The right side B of each example illustrates results obtained by performing the estimation method according to the disclosure for determining at least the model of the cardiac implant. The results of the computational analysis can be seen as a ground truth. The results obtained by performing the method according to the disclosure are very similar to the ground truth results and can be determined in a significantly more efficient way. The method and system according to the disclosure provides for a real-time determination/estimation of the models of the cardiac implant, without requiring computational intensive analysis (e.g. finite element analysis).

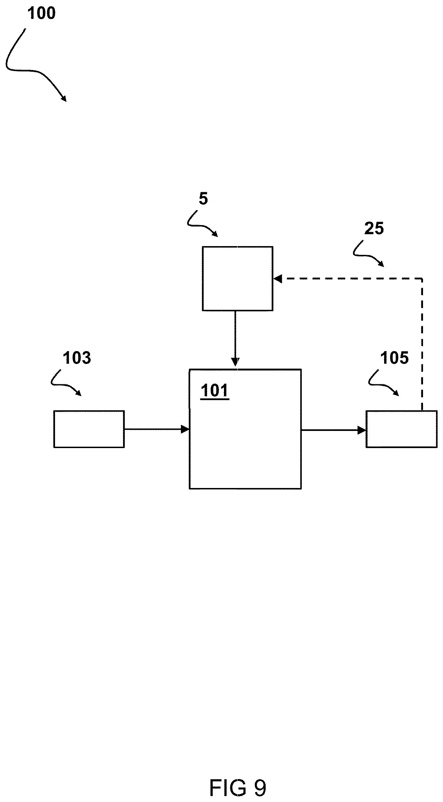

Fig. 9 shows a schematic diagram of an embodiment of a system 100 for determining a model 3 of a cardiac implant deployed in a cardiac region. The system includes a database 5 comprising a plurality of different records 7a-7N, each record including data representative of an image 9 of a cardiac region and an associated model 11 of a cardiac implant deployed in said cardiac region. The system 100 also comprises a processing unit 101 configured to: receive a patient-specific image 13 of the cardiac region for which the model 3 of the cardiac implant is to be determined; calculate an approximation of the patient-specific image 3 of the cardiac region based on a weighted combination of images 11 of the cardiac region in a plurality of records 7a-7N; and determine the model 3 of the cardiac implant deployed in the cardiac region associated to the patient-specific image 13 of the cardiac region based on weighted combination of models 11 associated to the images 9 of the cardiac region in the plurality of records 7a-7N used for approximating said patient-specific image 13 of the cardiac region.

In some examples, the system 100 comprises a receiving unit 103 which is configured to receive the patient-specific image 13 of the cardiac region for which the model 3 of the cardiac region is to be determined. In some examples, the receiving unit 103 may be configured to receive the image 13 from another device. The image 13 may be obtained by means of a medical imaging device (e.g. CT system, MRI system, PET system, and/or e.g. hybrid imaging systems such PET-MRI, PET-CT, etc.). In some examples, the receiving unit 103 is communicatively coupled to the medical imaging device. However, it is also envisaged that the receiving unit 103 receives the image via a database, the cloud, or by other means. The patient-specific image 13 may be a (pre-)processed image 13, for example obtained by performing one or more (pre-)processing steps. The system 100 may have an outputting unit 105, which can output the calculated model 3 of the cardiac region associated to the patient-specific image 13 of the cardiac region. Although the receiving unit 103 and the outputting unit 105 are drawn separately, they can also be a part of the processing unit 101. In some examples, the determined model 13 of the cardiac region is stored in the same database 5 (shown via line 25) or different database (not shown) and usable for determining subsequent patient-specific three-dimensional models.

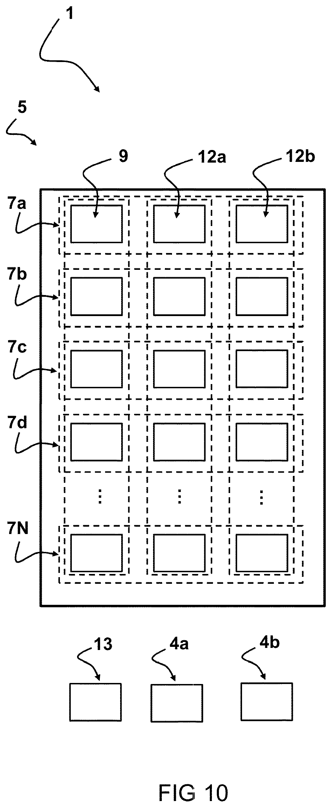

Fig. 10 shows a schematic diagram of an embodiment of a computer implemented method 1. A schematic representation of the database 5 is shown with a plurality of records 7a-7N. The records may be patient-specific. For example, each record may be associated to a single patient. In this example, each record includes a model 12b of the cardiac region with a cardiac implant deployed in the cardiac region. In addition, each record also includes a model 12a of the cardiac region without a cardiac implant deployed in the cardiac region. The model 4b of the cardiac region with implant associated to the patient-specific image of the cardiac region is determined based on weighted combination of models 12b with cardiac implant associated to images 9 of cardiac regions in the plurality of records 7a-7N used for approximating the patient-specific image 13 of the cardiac region. Additionally or alternatively, it is also possible to similarly determine the model 4a of the cardiac region without implant associated to the patient-specific image 13 of the cardiac region.

Therefore, instead of making a model of only the cardiac region, it is also possible to make a model with an implant (e.g. not present in the initial patient-specific 3D image).

In some examples, the 3D images 9 are without any implant. However, data of the model with implant may be available in the records 7a-7N of the database 5. Based on this data, it is possible to calculate a model with implant by performing a weighted combination, wherein the weighted combination is determined by first approximating the patient-specific image 13 using the 3D images 9 in the database. In some examples, a linear combination of 3D models can be made based on the relationship of the CT volumes. Based on this relationship, it is possible to make a combination of respective models with implants in order to build or approximate the model 4b with cardiac implant.

In some examples, the predicting can include querying a database including a plurality of records, each record including data representative of an image of a cardiac region and an associated size, and optionally position, of the cardiac implant in the cardiac region. The record can include data representative of the predefined criterion. The record can for instance include a medical image of a cardiac region. The record can include a model, such as a finite element model, representative of a cardiac region.

Optionally, the database includes records, each associated with a respective patient-specific clinical data. The database can include a plurality of medical images of cardiac regions of respective patients. The records can include data representative of implants implanted in the cardiac regions of the respective patients. The records can include data representative of the predetermined criterion. The database can include a plurality of models, such as finite element models, representative of cardiac regions of respective patients. Alternatively, or additionally, the database includes records associated with simulated data. The simulated data can be simulations of medical images of cardiac regions of respective patients. The simulated data can be models, such as a finite element models, representative of cardiac regions of respective patients.

It will be further understood that when a particular step of a method is referred to as subsequent to another step, it can directly follow said other step or one or more intermediate steps may be carried out before carrying out the particular step, unless specified otherwise. Likewise it will be understood that when a connection between components is described, this connection may be established directly or through intermediate components, unless specified otherwise or excluded by the context.

It will be appreciated that the method may include computer implemented steps. All above mentioned steps can be computer implemented steps. Embodiments may comprise computer apparatus, wherein processes performed in computer apparatus. The invention also extends to computer programs, particularly computer programs on or in a carrier, adapted for putting the invention into practice. The program may be in the form of source or object code or in any other form suitable for use in the implementation of the processes according to the invention. The carrier may be any entity or device capable of carrying the program. For example, the carrier may comprise a storage medium, such as a ROM, for example a semiconductor ROM or hard disk. Further, the carrier may be a transmissible carrier such as an electrical or optical signal which may be conveyed via electrical or optical cable or by radio or other means, e.g. via the internet or cloud.

Some embodiments may be implemented, for example, using a machine or tangible computer-readable medium or article which may store an instruction or a set of instructions that, if executed by a machine, may cause the machine to perform a method and/or operations in accordance with the embodiments.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips, chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

Herein, the invention is described with reference to specific examples of embodiments of the invention. For the purpose of clarity and a concise description features are described herein as part of the same or separate embodiments, however, alternative embodiments having combinations of all or some of the features described in these separate embodiments are also envisaged and understood to fall within the framework of the invention as outlined by the claims. The specifications, figures and examples are, accordingly, to be regarded in an illustrative sense rather than in a restrictive sense. The invention is intended to embrace all alternatives, modifications and variations which fall within the scope of the appended claims. Further, many of the elements that are described are functional entities that may be implemented as discrete or distributed components or in conjunction with other components, in any suitable combination and location.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality.

## Claims

1. A computer implemented method for determining a model of a cardiac implant deployed in a cardiac region of a patient, the method including:
providing a database including a plurality of different records, each record including at least data representative of an image of a cardiac region and an associated model of the cardiac implant deployed in said cardiac region;
receiving a patient-specific image of the cardiac region for which the model of the cardiac implant deployed in said cardiac region is to be determined;
calculating an approximation of the patient-specific image of the cardiac region based on a weighted combination of images of the cardiac region in a plurality of records, wherein the approximation is performed by means of an approximation function which is configured to approximate the patient-specific image of the cardiac region based on a weighted sum of data indicative of images of the cardiac region in a plurality of records; and
determining the model of the cardiac implant deployed in the cardiac region associated to the patient-specific image of said cardiac region based on parameters of the approximation function.

2. The computer implemented method according to claim 1, wherein the weighted combination is a linear combination.

3. The computer implemented method according to claim 1 or 2, wherein the approximation of the patient-specific image of the cardiac region is based on the weighted combination of images of the cardiac region of a selected subset of records.

4. The computer implemented method according to claim 3, wherein the subset of records is selected based on its weighted contribution for approximation of the image of the cardiac region.

5. The computer implemented method according to claim 1 or 2, wherein the approximation of the patient-specific image of the cardiac region is based on the weighted combination of images of the cardiac region of all the records in the database.

6. The computer implemented method according to any of the preceding claims, wherein each record includes at least one model of the cardiac region with a cardiac implant deployed in the cardiac region, wherein the model of the cardiac region with implant associated to the patient-specific image of the cardiac region is determined based on weighted combination of models with cardiac implant associated to images of cardiac regions in the plurality of records used for approximating the patient-specific image of the cardiac region.

7. The computer implemented method according to any of the preceding claims, wherein the models of the cardiac implant in the different records represent the same cardiac implant deployed in cardiac regions of different patients, wherein an undeformed cardiac implant is identical for all the records.

8. The computer implemented method according to claim 6 or 7, wherein the cardiac implant is at least one of a heart valve, a stent or an implantable medical device.

9. The computer implemented method according to any of the preceding claims, wherein the determined model of the cardiac implant deployed in the cardiac region is stored in the database and usable for determining subsequent patient-specific cardiac implant models.

10. The computer implemented method according to any of the preceding claims, wherein the models are at least one of a computer model, a geometrical model, a meshed model or a parametric model.

11. The computer implemented method according to any of the preceding claims, wherein the images of the cardiac region are pre-processed, wherein the pre-processing includes segmentation.

12. A system for determining a model of a cardiac implant deployable in a cardiac region, the system including:
a database comprising a plurality of different records, each record including data representative of an image of a cardiac region and an associated model of the cardiac implant deployed in said cardiac region; and
a processing unit configured to:
receive a patient-specific image of the cardiac region for which the model of the cardiac implant deployed in said cardiac region is to be determined;
calculate an approximation of the patient-specific image of the cardiac region based on a weighted combination of images of the cardiac region in a plurality of records, wherein the approximation is performed by means of an approximation function which is configured to approximate the patient-specific image of the cardiac region based on a weighted sum of data indicative of images of the cardiac region in a plurality of records; and
determine the model of the cardiac implant deployable in the cardiac region associated to the patient-specific image of said cardiac region based on parameters of the approximation function.

13. A computer implemented method for determining a model of a cardiac region, the method including:
providing a database including a plurality of different records, each record including at least data representative of an image of a cardiac region and an associated model of said cardiac region;
receiving a patient-specific image of the cardiac region for which the model of the cardiac region is to be determined;
calculating an approximation of the patient-specific image of the cardiac region based on a weighted combination of images of the cardiac region in a plurality of records, wherein the approximation is performed by means of an approximation function which is configured to approximate the patient-specific image of the cardiac region based on a weighted sum of data indicative of images of the cardiac region in a plurality of records; and
determining the model of the cardiac region associated to the patient-specific image of the cardiac region based on parameters of the approximation function.

14. A computer program product configured for performing, when run on a controller, the steps of the method according to any of claims 1-11 and 13.

## Patentansprüche

1. Computerimplementiertes Verfahren zur Bestimmung eines Modells eines Herzimplantats, das in einer Herzregion eines Patienten eingesetzt ist, wobei das Verfahren folgende Schritte einschließt:
Bereitstellen einer Datenbank, einschließlich einer Vielzahl verschiedener Datensätze, wobei jeder Datensatz zumindest Daten einschließt, die ein Bild einer Herzregion und ein zugehöriges Modell des Herzimplantats darstellen, das in der Herzregion eingesetzt ist;
Empfangen eines patientenspezifischen Bildes der Herzregion, für die das Modell des in der Herzregion eingesetzten Herzimplantats bestimmt werden soll;
Berechnen einer Approximation des patientenspezifischen Bildes der Herzregion basierend auf einer gewichteten Kombination von Bildern der Herzregion in einer Vielzahl von Datensätzen, wobei die Approximation mittels einer Approximationsfunktion vorgenommen wird, die konfiguriert ist, um das patientenspezifische Bild der Herzregion basierend auf einer gewichteten Summe von Daten zu approximieren, welche Bilder der Herzregion in einer Vielzahl von Datensätzen darstellen; und
Bestimmen des Modells des in der Herzregion eingesetzten Herzimplantats, das dem patientenspezifischen Bild der Herzregion zugeordnet ist, und zwar basierend auf den Parametern der Approximationsfunktion.

2. Computerimplementiertes Verfahren nach Anspruch 1, wobei die gewichtete Kombination eine Linearkombination ist.

3. Computerimplementiertes Verfahren nach Anspruch 1 oder 2, wobei die Approximation des patientenspezifischen Bildes der Herzregion auf der gewichteten Kombination von Bildern der Herzregion einer ausgewählten Teilmenge von Datensätzen basiert.

4. Computerimplementiertes Verfahren nach Anspruch 3, wobei die Teilmenge von Datensätzen basierend auf ihrem gewichteten Beitrag zur Approximation des Bildes der Herzregion ausgewählt wird.

5. Computerimplementiertes Verfahren nach Anspruch 1 oder 2, wobei die Approximation des patientenspezifischen Bildes der Herzregion auf der gewichteten Kombination der Bilder der Herzregion aller Datensätze in der Datenbank basiert.

6. Computerimplementiertes Verfahren nach einem der vorstehenden Ansprüche, wobei jeder Datensatz mindestens ein Modell der Herzregion mit einem in der Herzregion eingesetzten Herzimplantat einschließt, wobei das Modell der Herzregion mit dem Implantat, das dem patientenspezifischen Bild der Herzregion zugeordnet ist, und zwar basierend auf einer gewichteten Kombination von Modellen mit einem Herzimplantat, die Bildern von Herzregionen in der Vielzahl von Datensätzen zugeordnet sind, die zur Approximation an des patientenspezifischen Bildes der Herzregion verwendet werden.

7. Computerimplementiertes Verfahren nach einem der vorstehenden Ansprüche, wobei die Modelle des Herzimplantats in den verschiedenen Datensätzen dasselbe Herzimplantat darstellen, das in Herzregionen verschiedener Patienten eingesetzt wird, wobei ein unverformtes Herzimplantat für alle Datensätze identisch ist.

8. Computerimplementiertes Verfahren nach Anspruch 6 oder 7, wobei das Herzimplantat mindestens eine Herzklappe, ein Stent oder eine implantierbare medizinische Vorrichtung ist.

9. Computerimplementiertes Verfahren nach einem der vorstehenden Ansprüche, wobei das bestimmte Modell des in der Herzregion eingesetzten Herzimplantats in der Datenbank gespeichert wird und zur Bestimmung nachfolgender patientenspezifischer Herzimplantatmodelle verwendbar ist.

10. Computerimplementiertes Verfahren nach einem der vorstehenden Ansprüche, wobei die Modelle mindestens eines der folgenden Modelle sind: ein Computermodell, ein geometrisches Modell, ein Netzmodell oder ein parametrisches Modell.

11. Computerimplementiertes Verfahren nach einem der vorstehenden Ansprüche, wobei die Bilder der Herzregion vorverarbeitet werden, wobei die Vorverarbeitung eine Segmentierung einschließt.

12. System zur Bestimmung eines Modells eines Herzimplantats, das in einer Herzregion eingesetzt werden kann, wobei das System Folgendes einschließt:
eine Datenbank umfassend eine Vielzahl verschiedener Datensätze, wobei jeder Datensatz Daten einschließt, die ein Bild einer Herzregion und ein zugehöriges Modell des Herzimplantats darstellen, das in dieser Herzregion eingesetzt ist; und
eine Verarbeitungseinheit, die für Folgendes konfiguriert ist:
Empfangen eines patientenspezifischen Bildes der Herzregion, für die das Modell des in der Herzregion eingesetzten Herzimplantats bestimmt werden soll;
Berechnen einer Approximation des patientenspezifischen Bildes der Herzregion basierend auf einer gewichteten Kombination von Bildern der Herzregion in einer Vielzahl von Datensätzen, wobei die Approximation mittels einer Approximationsfunktion vorgenommen wird, die konfiguriert ist, um das patientenspezifische Bild der Herzregion basierend auf einer gewichteten Summe von Daten zu approximieren, welche Bilder der Herzregion in einer Vielzahl von Datensätzen darstellen; und
Bestimmen des Modells des in der Herzregion einsetzbaren Herzimplantats, das dem patientenspezifischen Bild der Herzregion zugeordnet ist, und zwar basierend auf den Parametern der Approximationsfunktion.

13. Computerimplementiertes Verfahren zur Bestimmung eines Modells einer Herzregion, wobei das Verfahren folgende Schritte einschließt:
Bereitstellen einer Datenbank, einschließlich einer Vielzahl verschiedener Datensätze, wobei jeder Datensatz zumindest Daten einschließt, die ein Bild einer Herzregion und ein zugehöriges Modell der Herzregion darstellen;
Empfangen eines patientenspezifischen Bildes der Herzregion, für die das Modell der Herzregion bestimmt werden soll;
Berechnen einer Approximation des patientenspezifischen Bildes der Herzregion basierend auf einer gewichteten Kombination von Bildern der Herzregion in einer Vielzahl von Datensätzen, wobei die Approximation mittels einer Approximationsfunktion vorgenommen wird, die konfiguriert ist, um das patientenspezifische Bild der Herzregion basierend auf einer gewichteten Summe von Daten zu approximieren, welche Bilder der Herzregion in einer Vielzahl von Datensätzen darstellen; und
Bestimmen des Modells der Herzregion, das dem patientenspezifischen Bild der Herzregion zugeordnet ist, und zwar basierend auf Parametern der Approximationsfunktion.

14. Computerprogrammprodukt, das konfiguriert ist, um bei Ausführung auf einem Steuergerät die Schritte des Verfahrens nach einem der Ansprüche 1 bis 11 und 13 durchzuführen.

## Revendications

1. Procédé mis en œuvre par ordinateur pour la détermination d'un modèle d'un implant cardiaque déployé dans une région cardiaque d'un patient, le procédé incluant :
la fourniture d'une base de données incluant une pluralité de différents enregistrements, chaque enregistrement incluant au moins des données représentatives d'une image d'une région cardiaque et un modèle associé de l'implant cardiaque déployé dans ladite région cardiaque ;
la réception d'une image spécifique au patient de la région cardiaque pour laquelle le modèle de l'implant cardiaque déployé dans ladite région cardiaque doit être déterminé ;
le calcul d'une approximation de l'image spécifique au patient de la région cardiaque sur la base d'une combinaison pondérée d'images de la région cardiaque dans une pluralité d'enregistrements, dans lequel l'approximation est réalisée au moyen d'une fonction d'approximation qui est configurée pour donner une approximation de l'image spécifique au patient de la région cardiaque sur la base d'une somme pondérée de données indicatives d'images de la région cardiaque dans une pluralité d'enregistrements ; et
la détermination du modèle de l'implant cardiaque déployé dans la région cardiaque associé à l'image spécifique au patient de ladite région cardiaque sur la base de paramètres de la fonction d'approximation.

2. Procédé mis en œuvre par ordinateur selon la revendication 1, dans lequel la combinaison pondérée est une combinaison linéaire.

3. Procédé mis en œuvre par ordinateur selon la revendication 1 ou 2, dans lequel l'approximation de l'image spécifique au patient de la région cardiaque est basée sur la combinaison pondérée d'images de la région cardiaque d'un sous-ensemble sélectionné d'enregistrements.

4. Procédé mis en œuvre par ordinateur selon la revendication 3, dans lequel le sous-ensemble d'enregistrements est sélectionné sur la base de sa contribution pondérée à l'approximation de l'image de la région cardiaque.

5. Procédé mis en œuvre par ordinateur selon la revendication 1 ou 2, dans lequel l'approximation de l'image spécifique au patient de la région cardiaque est basée sur la combinaison pondérée d'images de la région cardiaque de tous les enregistrements dans la base de données.

6. Procédé mis en œuvre par ordinateur selon l'une quelconque des revendications précédentes, dans lequel chaque enregistrement inclut au moins un modèle de la région cardiaque avec un implant cardiaque déployé dans la région cardiaque, dans lequel le modèle de la région cardiaque avec implant associé à l'image spécifique au patient de la région cardiaque est déterminé sur la base d'une combinaison pondérée de modèles avec implant cardiaque associés à des images de régions cardiaques dans la pluralité d'enregistrements utilisés pour donner une approximation de l'image spécifique au patient de la région cardiaque.

7. Procédé mis en œuvre par ordinateur selon l'une quelconque des revendications précédentes, dans lequel les modèles de l'implant cardiaque dans les différents enregistrements représentent le même implant cardiaque déployé dans des régions cardiaques de différents patients, dans lequel un implant cardiaque non déformé est identique pour tous les enregistrements.

8. Procédé mis en œuvre par ordinateur selon la revendication 6 ou 7, dans lequel l'implant cardiaque est au moins l'un parmi une valvule du cœur, une endoprothèse ou un dispositif médical implantable.

9. Procédé mis en œuvre par ordinateur selon l'une quelconque des revendications précédentes, dans lequel le modèle déterminé de l'implant cardiaque déployé dans la région cardiaque est stocké dans la base de données et utilisable pour la détermination de modèles d'implant cardiaque spécifiques au patient ultérieurs.

10. Procédé mis en œuvre par ordinateur selon l'une quelconque des revendications précédentes, dans lequel les modèles sont au moins l'un parmi un modèle informatique, un modèle géométrique, un modèle maillé ou un modèle paramétrique.

11. Procédé mis en œuvre par ordinateur selon l'une quelconque des revendications précédentes, dans lequel les images de la région cardiaque sont prétraitées, dans lequel le prétraitement inclut une segmentation.

12. Système pour la détermination d'un modèle d'un implant cardiaque déployable dans une région cardiaque, le système incluant :
une base de données comprenant une pluralité de différents enregistrements, chaque enregistrement incluant des données représentatives d'une image d'une région cardiaque et un modèle associé de l'implant cardiaque déployé dans ladite région cardiaque ; et
une unité de traitement configurée pour :
recevoir une image spécifique au patient de la région cardiaque pour laquelle le modèle de l'implant cardiaque déployé dans ladite région cardiaque doit être déterminé ;
calculer une approximation de l'image spécifique au patient de la région cardiaque sur la base d'une combinaison pondérée d'images de la région cardiaque dans une pluralité d'enregistrements, dans lequel l'approximation est réalisée au moyen d'une fonction d'approximation qui est configurée pour donner une approximation de l'image spécifique au patient de la région cardiaque sur la base d'une somme pondérée de données indicatives d'images de la région cardiaque dans une pluralité d'enregistrements ; et
déterminer le modèle de l'implant cardiaque déployable dans la région cardiaque associé à l'image spécifique au patient de ladite région cardiaque sur la base de paramètres de la fonction d'approximation.

13. Procédé mis en œuvre par ordinateur pour la détermination d'un modèle d'une région cardiaque, le procédé incluant :
la fourniture d'une base de données incluant une pluralité de différents enregistrements, chaque enregistrement incluant au moins des données représentatives d'une image d'une région cardiaque et un modèle associé de ladite région cardiaque ;
la réception d'une image spécifique au patient de la région cardiaque pour laquelle le modèle de la région cardiaque doit être déterminé ;
le calcul d'une approximation de l'image spécifique au patient de la région cardiaque sur la base d'une combinaison pondérée d'images de la région cardiaque dans une pluralité d'enregistrements, dans lequel l'approximation est réalisée au moyen d'une fonction d'approximation qui est configurée pour donner une approximation de l'image spécifique au patient de la région cardiaque sur la base d'une somme pondérée de données indicatives d'images de la région cardiaque dans une pluralité d'enregistrements ; et
la détermination du modèle de la région cardiaque associé à l'image spécifique au patient de la région cardiaque sur la base de paramètres de la fonction d'approximation.

14. Produit programme d'ordinateur configuré pour la réalisation, lors de son exécution sur un dispositif de commande, des étapes du procédé selon l'une quelconque des revendications 1 à 11 et 13.
